# EUROPEAN PATENT APPLICATION

(11) **EP 2 050 755 A1**
(43) Date of publication of application: **22.04.2009**
(21) Application number: 07291284.3
(22) Date of filing: 19.10.2007
(51) Int. Cl.: C07F 9/655, C08F 10/00, C08F 4/619, C08F 4/603

(54) **Supported ionic liquids or activating supports**

(71) Applicant: TOTAL PETROCHEMICALS RESEARCH FELUY, 7181 Seneffe (Feluy) (BE); CENTRE NATIONAL DE LA RECHERCHE SCIENTIFIQUE (CNRS), 75016 Paris (FR)
(72) Inventor: El Kadib, Abdelkrim, 34000 Montpellier (FR); Molvinger, Karine, 34080 Montpellier (FR); Brunel, Daniel, 34090 Montpellier (FR); Prades, Floran, 1400 Nivelles (BE); Razavi, Abbas, 7000 Mons (BE)
(74) Representative: Roufosse, Micheline C.

(57) **Abstract**

The present invention relates to the covalent anchorage of non-coordinating anions on mineral supports to prepare supported ionic liquids and to their use as activating supports for the polymerisation of ethylene and alpha-olefins. The invention also discloses the concomitant covalent anchorage of zwitterionic systems containing both the non-coordinating anion and the counter cation parts of the ionic liquids and of the activating supports.

## Description

The present invention relates to the covalent anchorage of non-coordinating anions on mineral supports to prepare supported ionic liquids and to their use as activating agents for the polymerisation of ethylene and alpha-olefins. The invention also discloses the concomitant covalent anchorage of zwitterionic systems containing both the non-coordinating anion and the counter cation parts of the ionic liquids and of the activating agents.

It is known that perfluorinated borates can be covalently anchored on different supports such as for example polystyrene and silica. This was reported in US-A-5427991 or in US-A-5869723 or in EP-A-1359166 or in WO03/035708. The counter cations of the grafted perfluorinated borate were anilinium-type or trityl cations when these solid systems were used to activate catalyst components in the polymerisation of ethylene and alpha-olefins. In parallel, various works were reported in literature concerning the direct chemisorption of tris (pentafluorophenyl)borane on silica surface such as for example J. Tian, S. Wang, Y. Feng, J. Li, S. Collins, "Borane-functionalized oxide supports: development of active supported metallocene catalysts at low aluminoxane loading" in J. Mol. Catal. A: Chemical , 1999, 144, 137-150, or in M. Bochmann, G.J. Pindado, S.J. Lancaster, "The versatile chemistry of metallocene polymerisation catalysts: new developments in half-sandwich complexes and catalyst heterogenisation." in J. Mol. Catal. A: Chemical, 1999, 146, 179-190, or in S. Charoenchaidet, S. Chavadej, E. Gulari, "Borane-functionalized silica supports in situ activated heterogeneous zirconocene catalysts for MAO-free ethylene polymerisation" in J. Mol. Catal. A: Chemical, 2002, 167-177, 185, or in K. Musikabhumma, T.P. Spaniol, J. Okuda, "Ethylene polymerization with "constrained-geometry" titanium catalysts over borate-modified silica supports" in Macromol. Chem. Phys., 2002, 203, 115-121. This strategy additionally requires the presence of trityl or substituted ammonium (preferably dimethylanilinium) as countercations.

These catalytic systems using supported activators are less active than equivalent homogeneous systems and the polymer properties are thereby degraded.

A new generation of solid activating supports has been developed and is described for example in Marks (J. Am. Chem. Soc., 1998, 120, 13533): it concerns sulfated zircone particles or in McDaniel (WO-9960033, WO-0123433, WO-0123434, WO-0144309, WO-0149747 et US-A-6548441) or in Saudemont (FR-A-2765225). All these activators are solids wherein surface acid sites are responsible for the activation.

These acid sites are metals combined with halides such as fluor or chlorine; metals can be selected from aluminium, titanium, zirconium or nickel.

The equivalent species in homogeneous catalysis are very poor activating species.

Compounds such as dimethylaluminium fluoride (DMAF) are used as activators in combination with triethylaluminium for the stereospecific polymerisation of propylene with compounds of the metallocene family with low productivity as described by Zambelli (Macromolecules 1989, 22, 2186). They do not activate metallocene complexes.

On the other hand, ionic liquids are also actively investigated as alternative solvent media in many industrial applications such as synthesis, catalysis, separation, electrochemistry or nanoparticles stabilisation (*"*Ionic Liquids -Industrial Applications to Green Chemistry" ACS Symposium Series, Eds RD. Rogers and K.S. Seddon, American Chemical Society, Washington, DC, 2001 ; "Innovative Applications of Ionic Liquids as "Green Engineering Liquids" H. Zhao, Chem. Eng. Comm., 2006, 193, 1660-1677). Synthesis is described for example in K.R. Seedon, Kinetic Catal., 1996, 37, 693; or in J. Howarth, Tetrahedron Lett., 2000, 41, 6627; or in F. Zulfiquar, G. Tanaka, Green Chemistry, 2000, 2, 6627. Catalysis is described for example in "Catalytic reactions in ionic liquids" R. Sheldon, Chem. Commun., 2001, 2399-2407; or in J.J. Peng, Y.Q. Deng, New J. Chem.*,* or in *"*Ionic liquids in Catalysis" T. Welton, Coord. Chem. Rev., 2004, 248, 2459-2477. Separation is described for example in H. Qiu, S. Jiang, X. Liu, J. Chromatography A, 2006, 1103, 265-270; or in H. Qiu, S. Jiang, X. Liu, L. Zhao, J. Chromatography A, 2006, 1106, 46-50. Electrochemistry is described for example in J. Dupont, R.F. de Souza, P.A.Z. Suarez, Chem. Rev., 2002, 102, 3667-369. Nanoparticle stabilisation is described for example in "The use of imidazolium ionic liquids for the formation and stabilization of Ir⁰ and Rh⁰ nanoparticles: efficient catalysts for the hydrogenation of arenes" G.S. Fonseca, A.P. Umpierre, P.F.P. Fichtner, S. Teixeira, Chem. Eur.J. 2003, 9, 3263-3269.

Perfluoroalkyltrifluoroborates were also used as counter anions for ionic liquids, particularly to stabilise cyclic quaternary ammonium as disclosed for example in "Cyclic quaternary ammonium ionic liquids with perfluoroalkyltrifluoroborates: synthesis, characterization, and properties" Z.-B. Zhou, H. Matsumoto, K. Tatsumi, Chem. Eur. J., 2006, 12, 2196-2212. Until now, ionic liquids have been covalently anchored on silica surface by means of their cationic part using silica silylating reagent such as 1-methyl-3-(3-triethoxysilyl)-propylimidazolium cation as disclosed for example in WO2002098560, or in WO2001032308, or in M.H. Valkenberg, C. de Castro, W.F. Höelderich, Green Chemistry, 2002, 4, 88-93, or in C.P. Mehnert, R.A. Cook, N.C. Dispenziere, M. Afework, J. Am. Chem; Soc., 2002, 124, 12932.

The ionic liquids can also be anchored by their anionic part: this is achieved by codeposition of chloroaluminate species anion on silica with the 1-butyl-3-methylimidazolium (bmim) as counter cation.

N-[(trifluoromethyl)sulfony] trifluoromethanesulfonimide or bis-((perfluoromethyl)sulfonyl)imide (TFMSI) is currently used as counter anion in ionic liquids in order to impart to the resulting ionic liquid, particular physico-chemical properties. This is described for example in "Synthesis of 2,2-biimidazolium-based ionic liquids: use as a new reaction medium and ligand for palladium-catalyzed Suzuki cross-coupling reactions" J-C. Xiao, J.M. Shreeve, in J. Org. Chem., 2005, 70, 3072-3078, or in "Lithium ion conduction in a organoborate zwitterion-LiTFSI mixture" A. Narita, W. Shibayama, K. Sakamoto, T. Mizumo, N. Matsumi, H. Ohno, in Chem. Commun, 2006, 1926-1928.

One of the objectives of this invention is the use of heterogenised perfluorinated aryl borates covalently grafted on silica, as counter anions, to stabilise cations such as 1,3-dialkyl-imidazolium or 1-alkyl-pyridinium or related systems, in order prepare supported ionic liquids.

Another aim of the present invention is the co-anchorage of two parts of ionic liquids onto a support: the anion and the cation.

It is a further goal of the present invention to prepare supported ionic liquids from N[(perfluoroalkyl)sulfonyl] perfluoroalkane sulfonimide (PFSI) anions covalently anchored on mineral support and to use them as activating supports for the polymerisation of ethylene and alpha-olefins.

It is an aim of the present invention to prepare new species of activating support.

It is another aim of the present invention to prepare very active single site supported catalyst systems that do not require the use of methylaluminoxane.

It is also an aim of the present invention to prepare polymers that have regular grain size.

Any one of these aims is at least partially fulfilled by the present invention.

The present invention relates to the covalent anchorage of non-coordinative perfluorinated anions on mineral oxide support.

Accordingly, the present invention discloses the preparation of covalently anchored N[(perfluoroalkyl)sulfonyl]perfluoroalkanesulfonamide system as precursors for the preparation of supported ionic liquids that can be used as activating supports suitable for the polymerisation of ethylene and alpha-olefins.

Perfluoroalkanesulfonic acid-containing solids, generally called "Nafion"type-functionalised silica, have been previously reported by Harmer et al. ("Unique silane modified perfluorosulfonic acids as versatile reagents for new solid acid catalysts" M.A. Harmer, Q. Sun, M.J. Michalczyk, Z. Yang, in Chem. Commun., 1997, 1803-1804; or in "Novel mesoporous silica-perfluorosulfonic acid hybrids as strong heterogeneous Brönsted catalysts" D.J. Macquarrie, S.J. Taverner, M.A. Harmer, in Chem. Commun., 2005, 263-265) and by Corma et al. ("Single-step preparation and catalytic activity of mesoporous MCM-41 and SBA-15 silicas functionalized with perfluorosulfonic acid groups analogous to Nafion " M. Alvaro, A. Corma, D. Das, V. Fornes, H. Garcia, in Chem. Commun., 2004, 956-957; or in "Nafion" functionalized mesoporous MCM-41 silica shows high activity and selectivity for carboxylic acid esterification and Friedel-Crafts acylation reactions, M. Alvaro, A. Corma, D. Das, V. Fornes, H. Garcia, J. Catal., 2005, 231, 48-55; or in WO 2005051540). They were used in supported acid catalysis such as electrophilic aromatic substitution reactions and fatty acid esterification.

Corma's supported perfluorosulfonic acid is the reaction product of silica surface on trifluoromethyl-1 trifluoro 1,2,2'- ethanesultone, said latter product being the reaction product of perfluoropropene and sulphur trioxide. Alternatively supported sulfonic acid can be obtained starting from perfluorinated alkan-1ene.

There are two types of supported perfluorinated sulfonic acids respectively represented in the schemes below as starting material 1 and starting material 2.

The present invention discloses a method for preparing supported ionic liquids that comprises the steps of:
a) reacting starting supported sulfonic acid 1 or 2 either with non-nucleophilic trimethylsilane in scheme (a) or with perfluroalkan-2-ethyltrialkoxysilane in scheme b) in order to make the sulfonic group environment hydrophobic 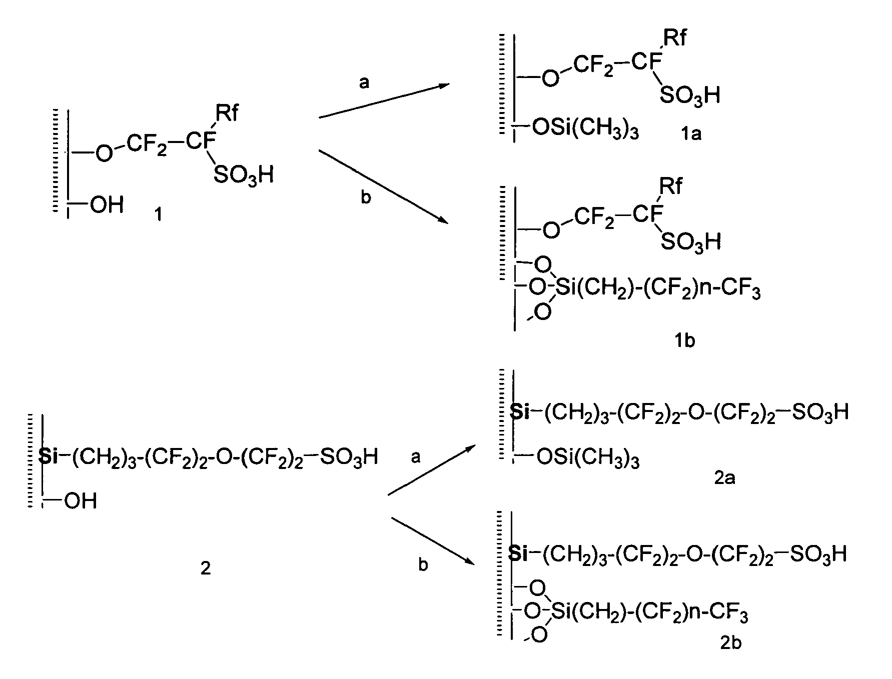 wherein Rf is perfluoroalkyl group CF₃(CF₂)ₙ- or (CF₃)₂CF(CF₂)ₙ- and wherein n is 0 or integer from 1 to 8;
b) reacting compounds 1, 1 a, 1b, 2a and 2b with thionylchloride to produce respectively supported perfluorosulfonic chloride products 1c, 1 d, 1 e, 2d and 2e 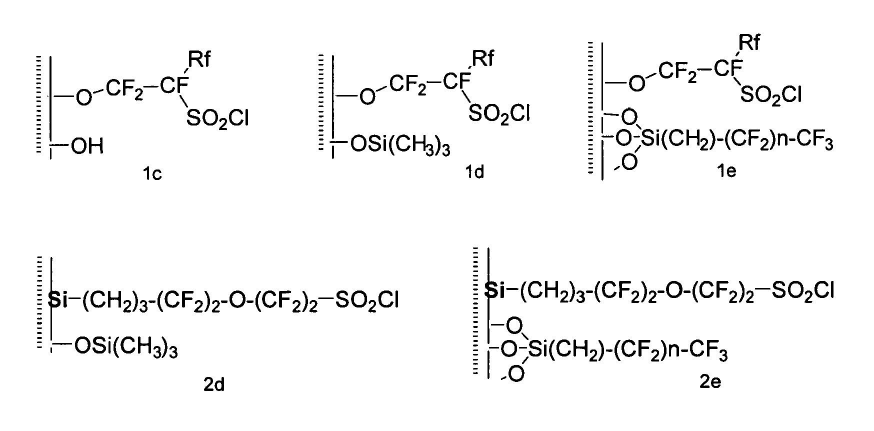
c) reacting perfluorosulfonyl chloride group-containing solids 1c, 1d, 1e, 2d, and 2e with ammonia to prepare perfluorosulfonamides compounds 1f, 1g, 1h, 2g and 2h 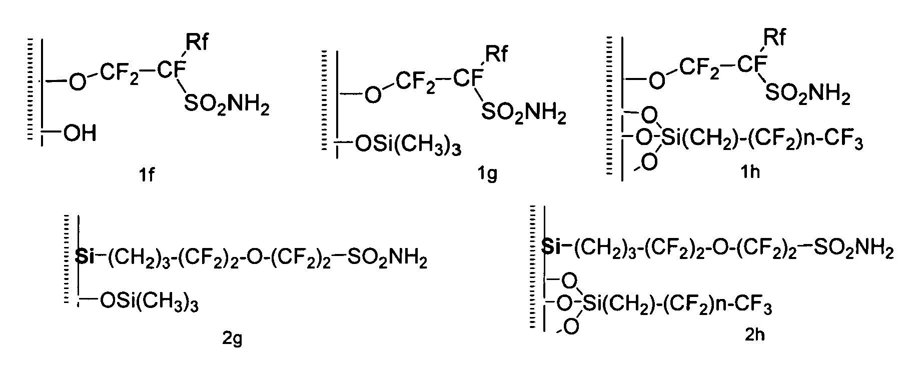
d) reacting perfluorosulfonamides compounds 1f, 1g, 1h, 2g and 2h with perfluoroalkanesulfonyl chloride Rf SO₂Cl in the presence of an organic base to prepare respectively the covalently anchored N[(perfluoroalkyl)sulfonyl] perfluoroalkane sulfonamide 1i, 1j, 1 k, 2j and 2k 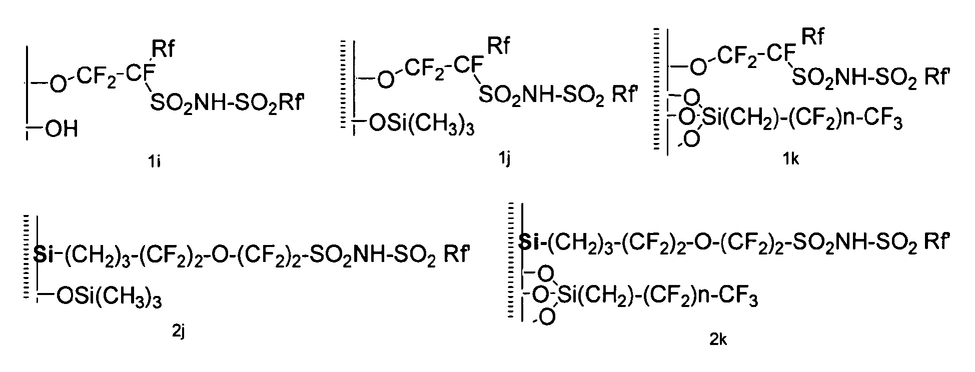 wherein Rf is perfluoroalkyl group CF₃(CF₂)ₙ- or (CF₃)₂CF(CF₂)ₙ-, wherein n is 0 or an integer from 1 to 8 and wherein Rf' is the same as or different from Rf.
e) reacting anchored N[(perfluoroalkyl)sulfonyl] perfluoroalkane sulfonamide 1i, 1j, 1 k, 2j and 2k with a metal hydroxide MOH or a metal organic derivative such as alkylate, arylate, aryl hydride or an organic base such as M-isopropyl amide or M-hexamethyldisilylazide, wherein M is a metal Group 1 of the periodic table, in order to obtain the corresponding covalently anchored N[(perfluoroalkyl)sulfonyl]-perfluoroalkane sulfonimide salts;
f) reacting the N[(perfluoroalkyl)sulfonylrperfluoroalkane sulfonimide salts-anchored supports of step e) with an organo-cation Cat, in order to obtain respectively compounds 1l, 1m, 1 n, 2m and 2n 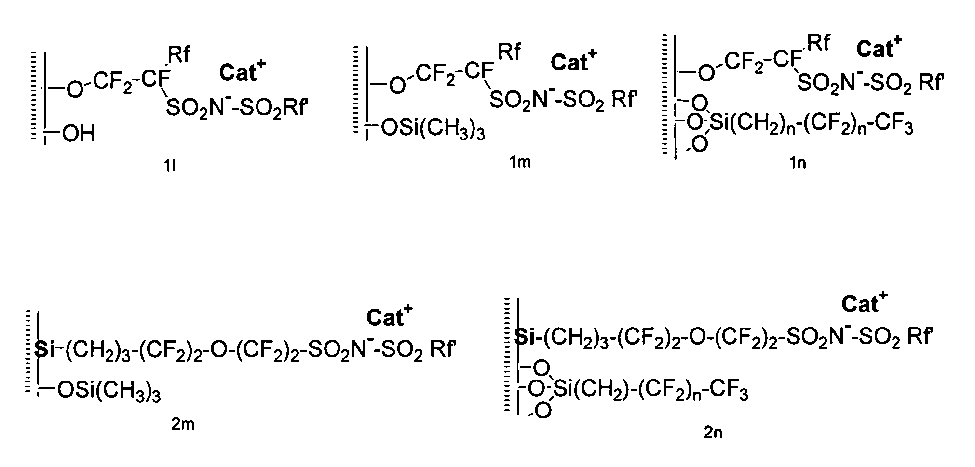
wherein Cat is trityl Ph₃CX or ammonium halide R₁R₂R₃R₄N⁺X⁻, wherein X is halogen, Ph is phenyl group, and R₁, R₂, R₃ and R₄ are each independently selected from hydrocarbons having from 1 to 6 carbon atoms.

Preferably at least one of R₁, R₂, R₃ and R₄ is aromatic group, more preferably phenyl group.

Preferably, M is selected from Li, Na, K. More preferably it is Li. The preferred metal organic derivative is lithium hexamethyldisilylazide: it offers the advantage of passivating the residual silanols by trimethylsilylation.

The preferred organic base of step d) is triethylamine.

Preferably, Rf and Rf are the same. More preferably they both are CF₃.

All supported ionic liquids according to the present invention can be used as activating supports, but not all activating supports are ionic liquids.
- When the cation is selected from trityl or aniline derivatives and the counter-anion is selected from halide or carboxylate, sulfonate, the resulting supported complex is an activating support suitable for the polymerisation of ethylene and alpha-olefins.
- When the cation is selected from dialkylimidazolium or 1-alkyl-pyridinium and related systems, the resulting complex is a supported ionic liquid.

In a preferred embodiment according to the present invention N-alkyl-N'-3-(propyltrialkoxysilyl) imidazolium is used as counter cation for co-grafting the cationic part of the ionic liquid system and PFSI is used as anionic part.

In another embodiment according to the present invention, N[(perfluoroalkyl)sulfonyl] perfluoroalkane sulfonimide compounds 1i, 1j, 1 k, 2j and 2k are synthesised in one step by reacting the perfluorosulfonyl chloride group-containing solids 1c, 1d, 1e, 2d and 2e of step b) with perflorosulfonamide RfSO₂NH₂ in presence of a solution of organic base.

In yet another embodiment according to the present invention, related to the supported perfluorosulfonic compounds of starting material 1, the reaction with an organo-cation of step f) is replaced by reaction with 1,3-diakyl-imidazolium bearing trialkoxysilyl groups (imid), that reacts with the residual hydroxyl groups of the mineral oxide surface to yield 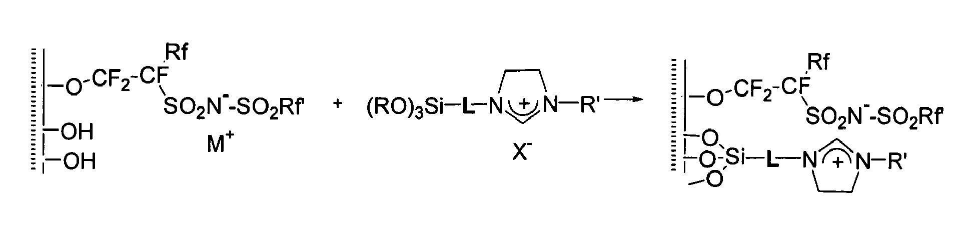 wherein linker group L is alkane or aryl group, R' is alkyl or aryl group, functionalised or not, and Rf and R'f are the same or different and are perfluoroalkyl groups CF₃(CF₂)ₙ- or (CF₃)₂CF(CF₂)ₙ- and wherein n is zero or an integer from 1 to 8.

The present invention also discloses the double anchorage of zwitterionic system performed by covalently anchoring both the anionic and the cationic parts of ionic liquids on a support.

A first embodiment discloses the preparation of supported ionic liquids by covalently anchoring perfluorinated arylborate anion and 1,3-diakyl-imidazolium cation by means of the trialkoxysilyl group present in each ionic part. 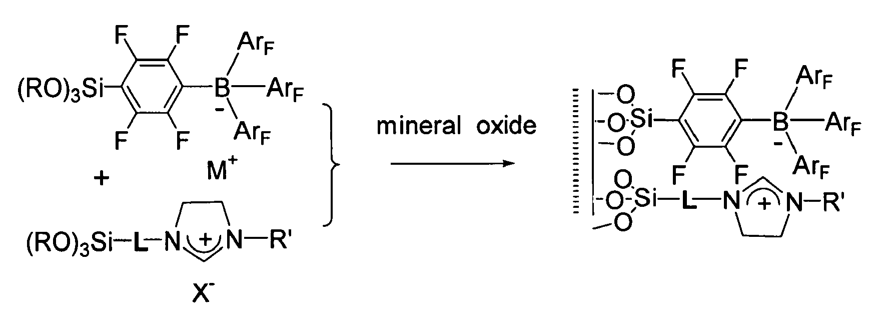 wherein Ar_{F} is perfluorinated aromatic nucleus, L is an alkane or aryl linker group, R is alkyl group and R' in functionalised or non-functionalised alkyl or aryl group.

In second embodiment discloses the co-anchorage on a mineral oxide support of N[(perfluoroalkyl)sulfonyl]-perfluoroalkane sulfonimide entities with imidazolium derivatives by means of the silylation of mineral oxide by zwitterionic species (PFSI⁻ ; imid⁺), wherein each component bears trialkoxysilane : 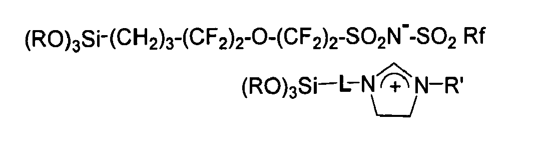 wherein R is alkyl group having from 1 to 6 carbon atoms and R' is selected from alkyl, aryl, alkylaryl or fluorinated chains optionally bearing functional groups such as sulfonic or phosphonic derivatives to yield

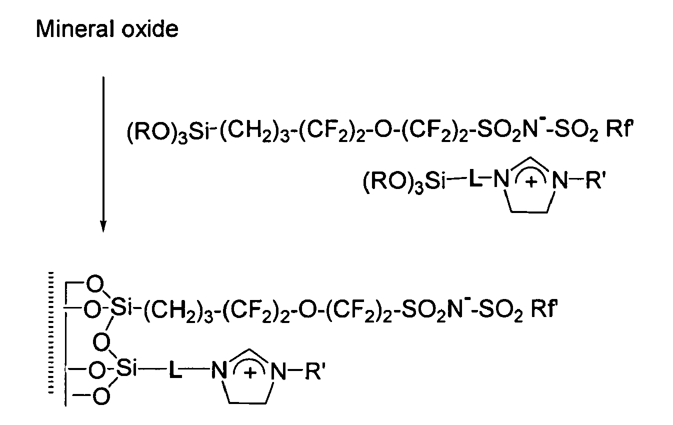

The support is preferably a silica support.

The present invention also discloses the activating supports and the supported ionic liquids obtainable by the method of the present invention.

These new supported ionic liquids and activating supports are suitable for preparing catalyst systems based on single site catalyst components. Metallocene or late transition metal complex catalyst components prepared by any method known in the art can be deposited on the supported ionic liquids or activating supports of the present invention in order to provide active catalyst systems without addition of conventional activating agents such as aluminoxanes or boron-based compounds. Late transition metal complexes of the present invention preferably include α-diimine Ni complexes as disclosed by Brookhart in W096/ 23010 or bis(imino)pyridyl Iron(II) or Cobalt(II) complexes as disclosed by Bristovsek et al. (G.J.P. Bristovsek, V.C. Gibson, B.S. Kimberley, P.J. Maddox, S.J. Mc Tavish, G.A. Solan, A.J.P. White, D.J.J. Williams, in Chem. Commun., 849-50, 1998) or in Small et al. (B.L. Small, M. Brookhart, A.M.A. Bennett, in J. Am. Chem. Soc., 120, 4049, 1998).

The metallocene component is preferably dialkylated. If it is dihalogenated, it is preferably used with an alkylating agent such as for example aluminium alkyl.

The present invention thus discloses a method for preparing an active catalyst system by the steps of:
a) providing a supported ionic liquid or an activating support as described in any one of the embodiments hereabove;
b) impregnating a metallocene or a post-metallocene catalyst component onto the activating support or supported ionic liquid;
c) optionally adding a scavenger;
d) retrieving an active catalyst system.

The active catalyst systems of the present invention are used for the oligomerisation or the homo- or co-polymerisation of ethylene and alpha-olefins. They have the advantage of being very efficient without the need to add costly and dangerous material such as methylaluminoxane.

The present invention thus discloses a method for oligomerising or homo- or co-polymerising that comprises the steps of:
a) injecting the active catalyst system of the present invention into the reactor;
b) injecting the monomer and optional comonomer simultaneously with or after the catalyst system;
c) optionally injecting a scavenger;
d) maintaining under polymerization conditions;
e) retrieving a polymer.

The monomer is preferably ethylene or propylene. The comonomer is preferably ethylene, propylene or 1-hexene.

### Examples.

The starting silica support used in all examples was G5H from Grace Davison with a surface area of 513 m².g⁻¹ and a porous volume of 1.8 mL.g⁻¹.

### Preparation of silica -grafted zwitterionic species-based (4-silyl-2,3,5,6-tetraflurophenyl)-tris(pentafluorophenyl)borate / 1-N-methyl-(3-silylorooyl)-3-N'-imidazolium.

In a first step, 0.7 g (2.26 mmol) of 1-methyl-3-(3-triethoxysilyl)-propylimidazolium chloride prepared according to Kumar et al [P. Kumar, W. Vermeiren, J-P. Dath, W.F. Höelderich, Appl. Catal, 2006, 304, 131-141] were added to a solution of Li (4-triethoxysilyl-2,3,5,6-tetrafluro-phenyl)-tris(pentafluorophenyl)borate (1.88g ; 2.26 mmol) in toluene (10mL). The solution containing the zwitterionic bis-silylated was then filtered to remove the lithium chloride.

2,16 g of Silica gel (G5H) were treated under vacuum at 150°C during 12h. After cooling under argon, the activated silica was suspended in toluene (10 mL), then 0.04 mmol of the same 1:4 mixture of trityl (4-triethoxysilyl-2,3,5,6-tetrafluoro-phenyl)tris(pentafluoro-phenyl) borate and phenyltrimethoxysilane, dissolved in toluene as that of the previous example, were poured into the stirred silica suspension under argon. The reaction mixture was stirred at room temperature during 3h, then heated at 60°C during 16 h. After cooling, the solid was filtered, under argon, then washed with anhydrous toluene (two times with 20 mL) then with pentane (two times with 20 mL) then evacuated under vacuum at room temperature during 4h.

### Preparation of 1,2,2-trifluoro-1-trifluoromethyl-ethane sulfonic acid-containing silica gel.

### Sample I.

A solution of 0.8 g (3.5 mmol) of 1,2,2-trifluoro-2-hydroxy1-(trifluoromethyl)-ethane sulfonic acid beta-sultone (from ABCR chemicals) in dry toluene (20mL) was added at room temperature to a suspension of 4 g of cooled activated silica gel ((G5H) (treated under vacuum at a temperature 150°C during 8h) in toluene (25mL) under argon. After stirring at room temperature during 30 min, the reaction mixture was heated at a temperature of 80°C during 12h then at a temperature of 100°C during 3h more. After cooling, the solid was filtered under argon and washed under argon with dry toluene (30mL, 2 times), then with pentane (20mL) then evacuated under vacuum 4h. Thermogravimetry analysis indicated a perfluorosulfonic loading of 0.2 mmol.g⁻¹

### Sample II.

Two other runs were carried out. In each run, 2.8 g of sultone (12 mmol) were contacted with 6g of activated silica using the same procedure. Thermogravimetry analysis indicated a perfluorosulfonic loading of 0.54 mmol.g⁻¹. Samples II

3.2 g of perfluorosulfonic acid -grafted silica of samples I and II were evacuated at a temperature of 100°C during 12h then each sample was treated with a solution of N,O-bis-trimethylsilyl-trifluoroacetamide (2 mL) in toluene (32 mL) and stirred. Each reaction mixture was heated at a temperature of 80°C during 12h. The passivated perfluorosulfonic acid -grafted silicas were filtered under argon, then washed with toluene (2 times 20 mL), pentane (2 times ,20mL) dichloromethane (2 times 20mL), then ethyl-ether (20 mL). The two passivated solids were then evacuated under vacuum.

### Preparation of passivated 1,2,2-trifluoro-1-trifluoromethyl-ethane sulfonyl chloride-containing silica gel.

3.2 g of each of the passivated sulfonic acid-grafted silica samples prepared hereabove were activated at a temperature of 100°C under vacuum during 12h. After cooling, 40mL of dry toluene were added under argon, followed by addition of freshly distilled SOCl₂ (4mL), corresponding to a large excess (4 equ.) of grafted sulfonic function. After stirring at room temperature during 30 min, the reaction mixtures were heated at a temperature of 110°C during 2h. After cooling, the excess SOCl₂ was evacuated under vacuum.

### Preparation of N-[(trifluoromethyl)sulfonyl]-(1,2,2-trifluoro-1-trifluoromethyl-ethane) sulfonamide-containing silica gel.

### First method: Preparation of passivated 1,2,2-trifluoro-1-trifluoromethyl-ethane sulfonamide-containing silica gel

3g of the passivated 1,2,2-trifluoro-1-trifluoromethyl-ethane sulfonyl chloride-containing silica gel obtained hereabove were poured in an autoclave (50mL) under a stream of argon. After cooling the autoclave at a temperature of -15°C, ammonia was admitted (5.5 bars) until the ammonia condensed weight was 6 g. The autoclave was gently returned to room temperature, the reaction mixture was maintained under stirring forr 3h30 at room temperature. Ammonia was then evacuated at normal pressure. The solid was washed with toluene (3 times , 30 mL), ethanol (30mL), ethanol-water mixture (2 times, 30mL), ethanol (20mL), filtered, then washed again with ethanol (20mL), pentane (2 times, 10 mL), then evacuated under vacuum.

### Preparation of passivated N-[(trifluoromethyl)sulfonyl]-1,2,2-trifluoro-1-trifluoromethyl-ethane) sulfonamide-containing silica gel.

To a suspension of passivated 1,2,2-trifluoro-1-trifluoromethyl-ethane sulfonamide-containing silica gel (1.5 g), previously activated at a temperature of 120°C during 6 h, then cooled under argon, in toluene (20mL), was added a solution of trifluoromethanesufonyl chloride (0.68g ; 4 mmol) and triethylamine (0.4 g; 4 mmol) in toluene (15 mL).

The reaction mixture was stirred at a temperature of 80 °C during 12h, then after cooling the solid was filtered , washed with toluene, methanol : water mixture (60:40), ethanol, ether, then with a refluxing dichloromethane: ethyl ether mixture (50:50) using a soxhlet apparatus. The solid was washed twice with pentane (30 mL) and dried under vacuum.

### Second method: Preparation of N-[(trifluoromethyl)sulfonyl]-(1,2,2-trifluoro-1-trifluoromethyl-ethane) sulfonamide-containing silica gel.

1,2,2-trifluoro-1-trifluoromethyl-ethane sulfonyl chloride-containing silica gel (1.6 g) was freshly prepared from 1,2,2-trifluoro-1-trifluoromethyl-ethane sulfonic acid-containing silica gel (sample III). It was prepared following the same procedure as that used to prepare sample II and contained ∼1mmol.g⁻¹: it was obtained by successive treatment with 4 mL of toluene and of SOCl₂ (3 mL, 25 mmol), at 100°C during 6h, then evacuation under vacuum during 4h. After cooling, the solid was suspended in toluene (25 mL) under argon and trifluoromethylsulfonamide (1g, 6.7 mmol, corresponding to 5 eq. vs sulfonyl chloride loading) was poured into the reaction mixture, then triethylamine (0.8 mL, corresponding to ∼5eq vs sulfonyl chloride loading) was injected with a syringe. The reaction mixture was heated at 90°C under stirring during 16h. After cooling, the solid was filtered, washed with toluene (50 mL, 3 times), washed with toluene, methanol : water mixture (60:40), ethanol, ether, then with a refluxing dichloromethane: ethyl ether mixture (50:50) using a soxhlet apparatus. The solid was washed twice with pentane (30 mL) and dried under vacuum.

### Preparation of trityl N-[(trifluoromethyl)sulfonyl]-(1,2,2-trifluoro-1-trifluoro-methyl-ethane) sulfonimide-containing silica gel.

In a first step, lithium hexamethyldisilazide was prepared by reaction of BuLi (2.5 mL, 0.3 10⁻² mol) in anhydrous THF (35 mL) with hexamethyldisilazane (0.65 mL, 0.3 10⁻² mol) at a temperature of -70°C under argon. The temperature was gently increased to -30°C. The solution was then poured dropwise into a stirred suspension of freshly activated N-[(trifluoromethyl)sulfonyl]-(1,2,2-trifluoro-1-trifluoromethyl-ethane) sulfonamide-containing silica gel (1.5 g; 0.3 10⁻³ mol of sulfonamide) in anhydrous THF (35 mL) at a temperature of 0°C. Then the temperature was allowed to return gently to room temperature and the reaction mixture was stirred during 1 h.

Then, a solution of trityl chloride (0.32 g, 1.2 mmol) in THF (20 mL) was added to the reaction mixture that was stirred at room temperature during 16h. The solid so obtained was filtered, under argon, then washed with anhydrous THF (twice with 30 mL), toluene (twice with 30mL) then with pentane (twice with 30mL), then evacuated under vacuum at room temperature during 4h.

The same procedure was applied to the passivated N-[(trifluoromethyl)sulfonyl]-(1,2,2-trifluoro-1-trifluoromethyl-ethane) sulfonamide-containing silica gel in order to prepare the passivated trityl N-[(trifluoromethyl)sulfonyl]-(1,2,2-trifluoro-1-trifluoromethyl-ethane) sulfonimide-containing silica.

### Preparation of zwitterionic 1-N-methyl-(3-silylpropyl)-3-N'-imidazolium. N-[(trifluoromethyl)sulfonyl]-(1,2,2-trifluoro-1-trifluoro-methyl-ethane) sulfonimide-containing silica gel.

A sample of non-passivated lithium N-[(trifluoromethyl)sulfonyl]-(1,2,2-trifluoro-1-trifluoromethyl-ethane) sulfonimide-containing silica (1.7 g) was suspended in THF (20 mL). Then a solution 1-methyl-3-(3-triethoxysilyl)-propylimidazolium chloride ( 0.35 g, 1.12 mmol) was poured under argon into the suspension. The reaction mixture was refluxed for 16h to undergo a condensation reaction of the silylated imidazolium derivatives on the residual silanols. The ionic liquid -supported silica was then filtered, then washed with (20 mL, twice), methanol : eau (2:1) mixture (20 mL, twice) in order to dissolve the lithium choride salts, then with methanol 100%, then with a mixture of dichloromethane : diethyl ether in a Soxhlet apparatus. The solid was washed twice with pentane (30 mL) and dried under vacuum.

### Poymerisation of ethylene.

The supported ionic liquids and activating supports prepared in the examples hereabove were used to polymerise ethylene under the following conditions:
Solvent: 20 mL of heptane
Scavenger: 1 mL of triisobutylaluminium (TIBAL)
Pressure: 15 bars of ethylene
Temperature: 50°C
Polymerisation time: 30 minutes
Stirring was carried out at 1000 rpm
Metallocene catalyst component: a fresh solution of dimethylated Finacene-Z (commercial Z12Hsm).
Activating support : 10 mg of 1,2,2-trifluoro-1-trifluoromethyl-ethane sulfonic acid-containing silica gel, passivated.
Ratio metallocene / activating support: 1.08.

Polyethylene was obtained with a productivity of 63 g/g/h and an activity of 0.34 kg/mmol/h. The morphology was good.

The polymerisation results are displayed in Table I.

## Claims

1. A method for preparing supported ionic liquids or activating supports that comprises the steps of:
a) reacting starting supported sulfonic acid 1 or 2 either with non-nucleophilic trimethylsilane in scheme (a) or with perfluroalkan-2-ethyltrialkoxysilane in scheme b) in order to make the sulfonic group environment hydrophobic 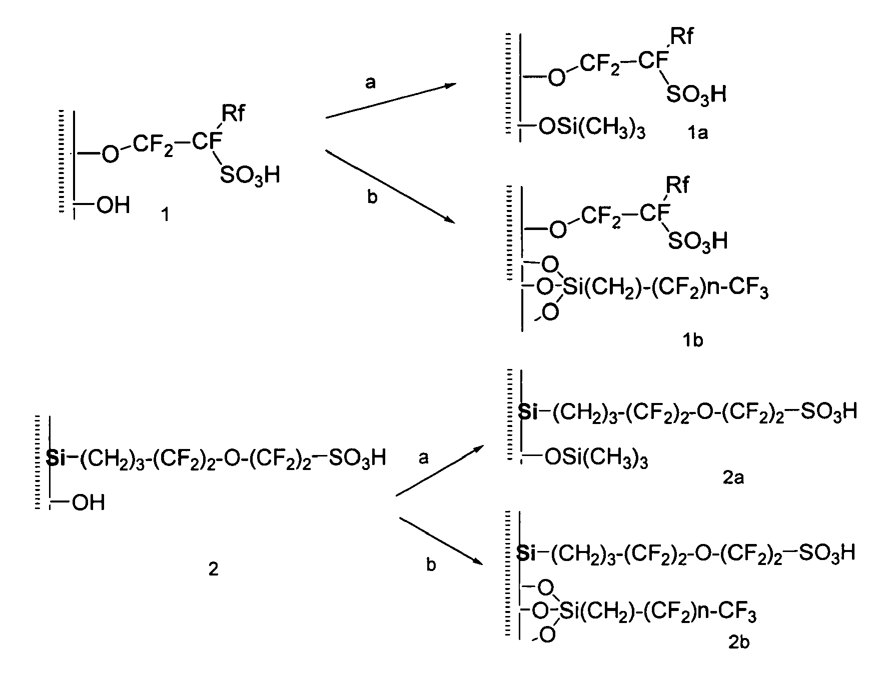 wherein Rf is perfluoroalkyl group CF₃(CF₂)ₙ- or (CF₃)₂CF(CF₂)ₙ- and wherein n is 0 or integer from 1 to 8;
b) reacting compounds 1, 1a, 1b, 2a and 2b with thionylchloride to produce respectively supported perfluorosulfonic chloride products 1c, 1d, 1e, 2d and 2e 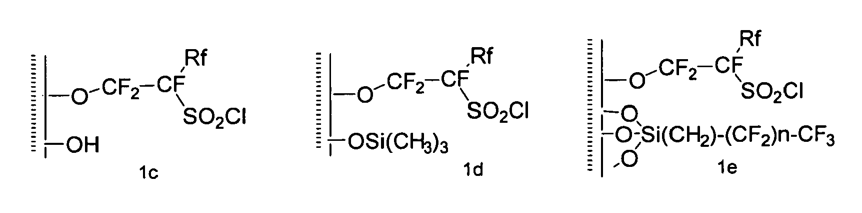 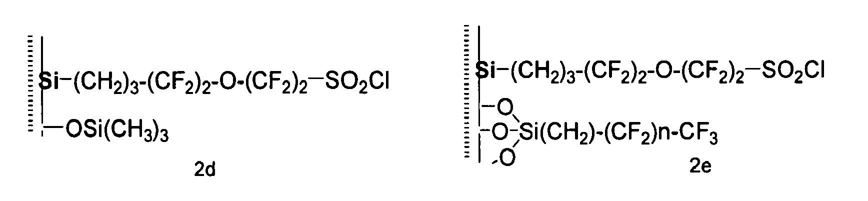
c) reacting perfluorosulfonyl chloride group-containing solids 1c, 1d, 1e, 2d, and 2e with ammonia to prepare perfluorosulfonamides compounds 1f, 1g, 1h, 2g and 2h 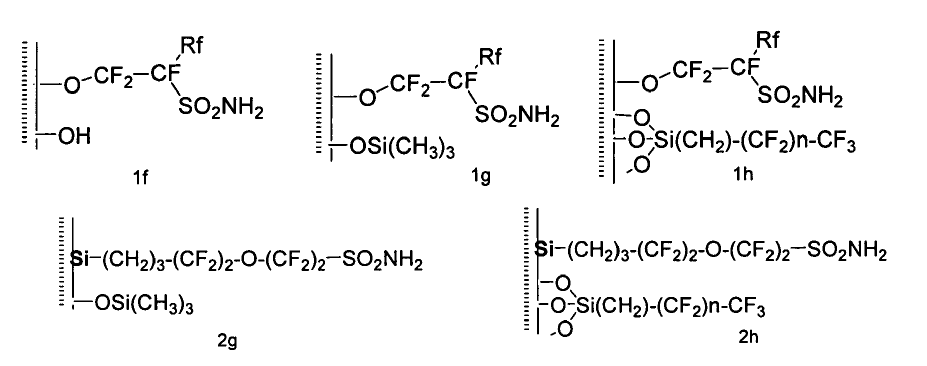
d) reacting perfluorosulfonamides compounds 1f, 1g, 1h, 2g and 2h with perfluoroalkanesulfonyl chloride Rf SO₂Cl in the presence of an organic base to prepare respectively the covalently anchored N[(perfluoroalkyl)sulfonyl] perfluoroalkane sulfonamide 1i, 1j, 1k, 2j and 2k 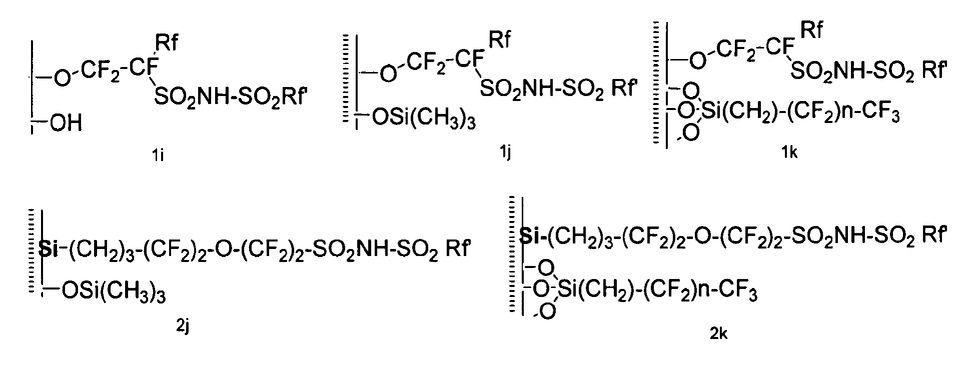 wherein Rf is perfluoroalkyl group CF₃(CF₂)ₙ- or (CF₃)₂CF(CF₂)ₙ-, wherein n is 0 or an integer from 1 to 8 and wherein Rf is the same as or different from Rf.
e) reacting anchored N[(perfluoroalkyl)sulfonyl] perfluoroalkane sulfonamide 1i, 1j, 1k, 2j and 2k with a metal hydroxide MOH or a metal organic derivative such as alkylate, arylate, aryl hydride or an organic base such as M-isopropyl amide or M-hexamethyldisilylazide, wherein M is a metal Group 1 of the periodic table, in order to obtain the corresponding covalently anchored N[(perfluoroalkyl)sulfonyl]-perfluoroalkane sulfonimide salts;
g) reacting the N[(perfluoroalkyl)sulfonyl]⁻perfluoroalkane sulfonimide salts-anchored supports of step e) with an organo-cation Cat, in order to obtain respectively compounds 1l, 1m, 1n, 2m and 2n 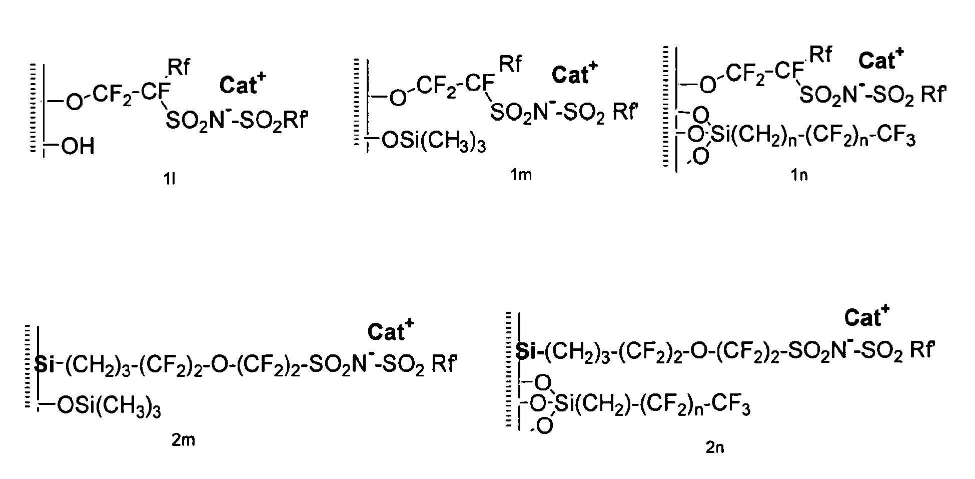 wherein Cat is trityl Ph₃CX or ammonium halide R₁R₂R₃R₄N⁺X⁻, wherein X is halogen, Ph is phenyl group, and R₁, R₂, R₃ and R₄ are each independently selected from hydrocarbons having from 1 to 6 carbon atoms.

2. The method of claim 1 wherein N[(perfluoroalkyl)sulfonyl] perfluoroalkane sulfonimide compounds 1i, 1j, 1k, 2j and 2k are synthesised in one step by reacting the perfluorosulfonyl chloride group-containing solids 1c, 1d, 1e, 2d and 2e of step b) with perflorosulfonamide RfSO₂NH₂ in presence of a solution of organic base.

3. A method for anchoring both anionic and cationic parts on the support wherein the anionic part is the perfluorosulfonic compound of starting material 1 of claim 1, reacted with 1,3-diakyl-imidazolium bearing trialkoxysilyl groups (imid), said trialkoxysilyl group reacting with the residual hydroxyl groups of the mineral oxide surface to anchor the cationic part on the support 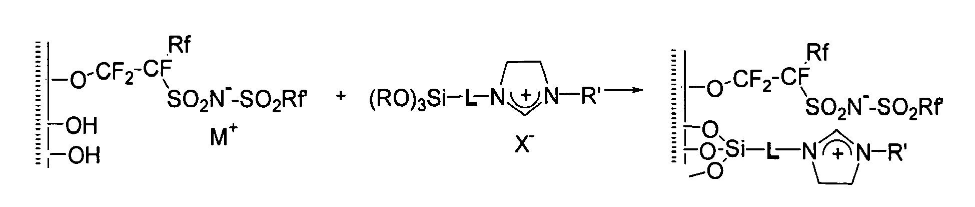 wherein linker group L is alkane or aryl group, R' is alkyl or aryl group, functionalised or not, and Rf and R'f are the same or different and are perfluoroalkyl groups CF₃(CF₂)ₙ- or (CF₃)₂CF(CF₂)ₙ- and wherein n is zero or an integer from 1 to 8.

4. The method of claim 3 wherein the anionic part is preplaced by perfluorinated arylborate anion to yield 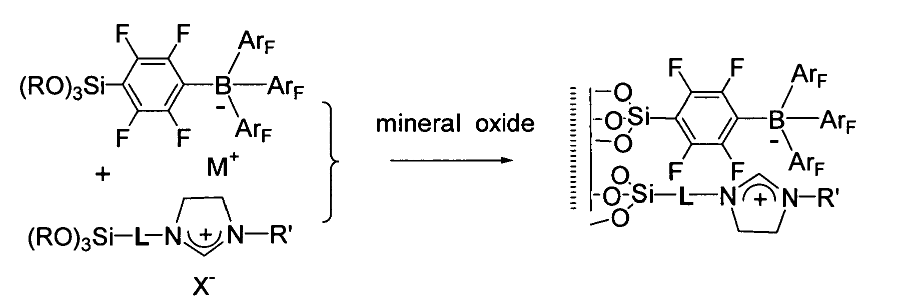 wherein Ar_{F} is perfluorinated aromatic nucleus.

5. The method of any one claims 1 to 3 wherein Rf and Rf' are the same.

6. The method of claim 5 wherein Rf and Rf' are both CF₃.

7. The method of any one of the preceding claims wherein M is Li.

8. The method of any one of the preceding claims wherein Cat is trityl or ammonium halide.

9. The supported ionic liquids and activating supports obtainable by the method of any one of claims 1 to 8.

10. A method for preparing an active catalyst system that comprises the steps of:
a) providing the supported ionic liquid or activating support of claim 9;
b) impregnating a metallocene or a post-metallocene catalyst component onto the activating support or supported ionic liquid;
c) optionally adding a scavenger;
d) retrieving an active catalyst system.

11. An active catalyst systems obtainable by the method of claim 10.

12. A method for oligomerising or homo- or co-polymerising ethylene and alpha-olefins that comprises the steps of:
a) injecting the active catalyst system of claim 11 into the reactor;
b) injecting the monomer and optional comonomer simultaneously with or after the catalyst system;
c) optionally injecting a scavenger;
d) maintaining under polymerisation conditions;
e) retrieving a polymer or oligomer.

13. The method of claim 12 wherein the monomer is selected from ethylene or propylene and the comonomer is selected from ethylene, propylene or 1-hexene.
